# EUROPEAN PATENT SPECIFICATION

(11) **EP 0 516 152 B1**
(45) Date of publication and mention of the grant of the patent: **27.03.1996**
(21) Application number: 92109105.4
(22) Date of filing: 29.05.1992
(51) Int. Cl.: A61M 1/34, A61M 1/16

(54) **Apparatus for hemodiafiltration treatment**
Vorrichtung zur Hämodiafiltrationsbehandlung
Dispositif d'hémodiafiltration

(30) Priority: 29.05.1991 IT BO910182
(43) Date of publication of application: 02.12.1992
(73) Proprietor: BELLCO S.p.A., I-41037 Mirandola(Modena) (IT)
(72) Inventor: Cianciavicchia, Domenico, 64040 Cavuccio (IT); Morselli, Massimo, 41038 S. Felice Sul Panaro (IT); Neri, Armando, 41033 Concordia Sul Secchia (IT); Pradelli, Alessandro, 41034 Finale Emilia (IT)
(74) Representative: Jorio, Paolo

(56) References cited:
- EP-A- 0 189 561
- EP-A- 0 212 127
- EP-A- 0 244 692
- EP-A- 0 441 328

## Description

The present invention relates to apparatus for hemodiafiltration treatment.

As is known, hemodiafiltration treatment involves a filtration of the patient's blood through a dialyser and, contemporaneously, but with a circuit separate from the filtration circuit, an infusion of dialysing liquid in the blood circuit downstream of the dialyser. The quantity of liquid which leaves the dialyser must be the sum of the quantity of fresh dialysing liquid which enters the dialyser and the quantity of water and metabolic waste withdrawn in the dialyser from the patient's blood. The said quantity of water and metabolic waste must in turn be the sum of the patient's set weight loss and a quantity equal to the quantity of infused liquid. In currently available apparatus the detection and management of the hydraulic balance just indicated is the most important problem in that it is necessary to have detection devices for each flow and in particular a first detection device for detecting the quantity of fresh dialysing liquid delivered by a pump to the dialyser, a second detector, normally constituted by an electronic balance, for detecting the quantity of infusion liquid delivered by a peristaltic pump, a third detector for detecting the quantity of liquid leaving the dialyser, and sometimes a fourth detector normally constituted by a balance for detecting the loss from the patient. These detection devices measure the absolute value of the quantities being measured whether these are weight, volume or mass flow rate, so that measurement errors caused by the type of detector utilised can arise here. These errors can add together and give rise to an incorrect hydraulic balance, which balance is essential for the good performance of the dialysing session since if the hydraulic balance is not regular the patient can manifest negative symptoms which sometimes are extremely dangerous, such as, for example, a cardio-circulatory collapse. It is therefore necessary to determine with absolute certainty that the quantity of liquid leaving the dialyser must be equal to the sum of the quantity of fresh dialysing liquid delivered to the dialyser, the programmed loss from the patient, and a quantity equal to that of the infusion liquid.

From the document EP-A-0 212 127, it is known an apparatus for hemodiafiltration having a dialyser and including a first circuit for the patient's body, having an input duct with a first pump and an output duct, and including a second circuit for circulating the dialysis solution in the dialyser, having an input duct with a second pump and an output duct, and wherein a branching duct branches from the input duct of the second circuit to supply fresh solution to the patient's body. The control of the second pump is effected by a pressure unit on the basis of the pressures sensed by some sensors into the ducts, which are processed according to an appropriate algorithm.

From the document EP-A-0 244 692, it is known a differential flow meter to the measurement of the amount of ultrafiltration occurring during a hemodialysis treatment, including a pair of tubes traversed by corresponding fluids in counter-current.

The object of the present invention is that of providing an apparatus for hemodiafiltration treatment which will be free from the stated disadvantages.

According to the invention there is provided an apparatus for hemodiafiltration treatment as defined by claim 1.

For a better understanding of the present invention a preferred embodiment is now described, purely by way o non-limitative example, with reference to the attached drawings, in which:
Figure 1 is a block schematic diagram of apparatus formed according to the principles of the present invention; and
Figure 2 is a front view of a component of the apparatus of Figure 1.

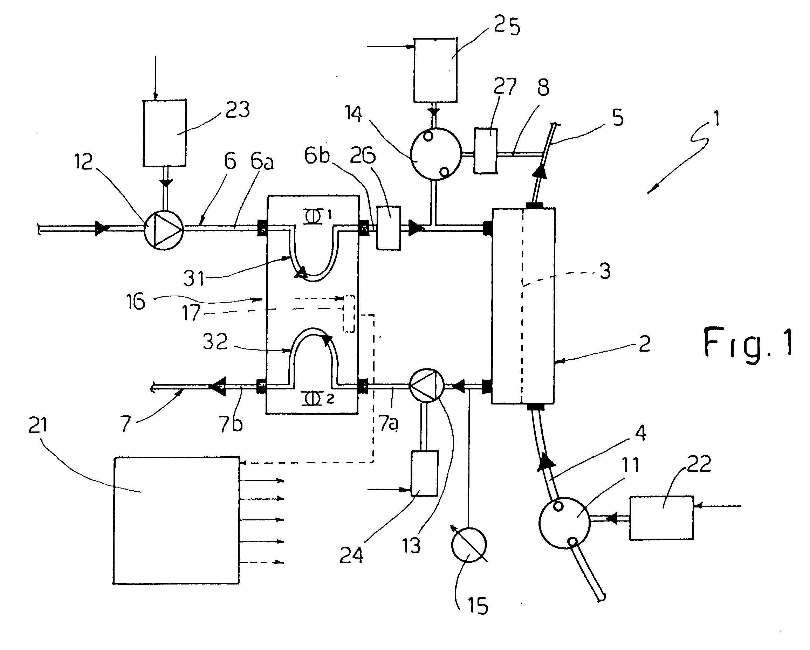

As illustrated in Figure 1 apparatus for hemodiafiltration treatment is generally indicated with the reference numeral 1 and comprises:
a dialyser 2 which as is known has a membrane 3 which separates a first extra-corporeal blood circuit defined by an input duct 4 and an output duct 5, from a second circuit in which the dialysing liquid circulates;
a duct 6 which forms part of the said second circuit in which the fresh dialysing liquid circulates, and which leads to the dialyser 2;
a duct 7 which forms part of the second circuit and which extends from the dialyser 2;
a duct 8 branching from the duct 6 and which joins the duct 5;
a pump 11 installed in the duct 4;
a pump 12 installed in the duct 6 upstream from the branching point of the duct 8;
a pump 13 installed in the duct 7 immediately downstream of the dialyser 2;
a pump 14 installed in the duct 8;
a device 15 operable to measure the value of the pressure in the duct 7 upstream of the pump 13;
a differential flow meter 16 installed in the duct 6 between the pump 12 and the branching point of the duct 8 and in the duct 7 downstream of the pump 13;
an electric unit 17 defined in the differential flow meter 16 and representative both of an electromagnet 18 and a device for generating an electrical signal proportional to the mass flow rate difference between the flows which traverse the flow meter 16; and
an electronic central control unit 21 operable to process the signal generated by the unit 17 and to control the pump 13.

By means of the associated motors 22, 23, 24 and 25 the central control unit 21 operates to control the pumps 11, 12, 13 and 14. Preferably, the pumps 11 and 14 are of peristaltic type. In use the quantities of infusion liquid and fresh dialysing liquid delivered to the dialyser 2, the quantity of blood put into circulation, and the hourly loss from the patient are decided by the medical operative on the basis of the patient's conditions. The apparatus 1 further includes two filters 26 and 27, the first installed in the duct 6 between the flow meter 16 and the branching point of the duct 8, and the second installed in the duct 8 downstream of the pump 14.

The differential flow meter is a device which detects the mass flow rate difference between two flows and which generates an electrical signal proportional to this difference. Essentially the differential flow meter has two tubes of small diameter through which flows a fluid in counter current. These tubes are similar to one another, have the same longitudinal extent and, by means of a crossover between respective central portions are made to lie in substantially the same plane. By means of an electromagnet acting on the crossover zone the assembly comprising the two tubes is made to oscillate at the resonant frequency of the overall assembly. If the flow rates along the tubes are the same the oscillation of the central portions takes place in the same plane. If the flow rates are different the so-called Coriolis forces due to the Coriolis principle cause an inclination of the central portions with respect to the said plane. The angle of inclination is proportional to the difference in the flow rates so that by measuring this angle it is possible to generate a corresponding signal.

Two different structures of differential flow meter are described in the European Patent Applications EP-A-0 244 692 and EP-A-0 441 328. These applications have been filed by the same applicant as the applicant for the present invention

In use the data relating to the quantity of fresh dialysing liquid which is delivered to the dialyser 2 and the quantity of this liquid which infuses into the patient's blood is set into the central control unit 21. On the basis of this data the central control unit suitably controls the pumps 12 and 14 in such a way that the sum of these quantities flows in the first tube of the flow meter 16. Data relating to the quantity of blood put into circulation by the pump 11 is also set into the central control unit 21. Finally, the patient's programmed loss, that is to say the imbalance between the mass flow rates of the fluids which flow in the flow meter is set into the central control unit 21 in such a way as to determine the value of the electrical signal generated by the unit 17 relating to this imbalance. In this way the central control unit 21, on the basis of the electrical signal from the unit 17, can drive the pump 13 in such a way that a quantity of fluid which determines the set imbalance flows along the second tube of the flow meter 16. It will appear evident that in the apparatus 1 the difference between the flows is evaluated not the absolute value of these and therefore, in the flow meter 16, a difference is evaluated with an intrinsic precision in this latter device.

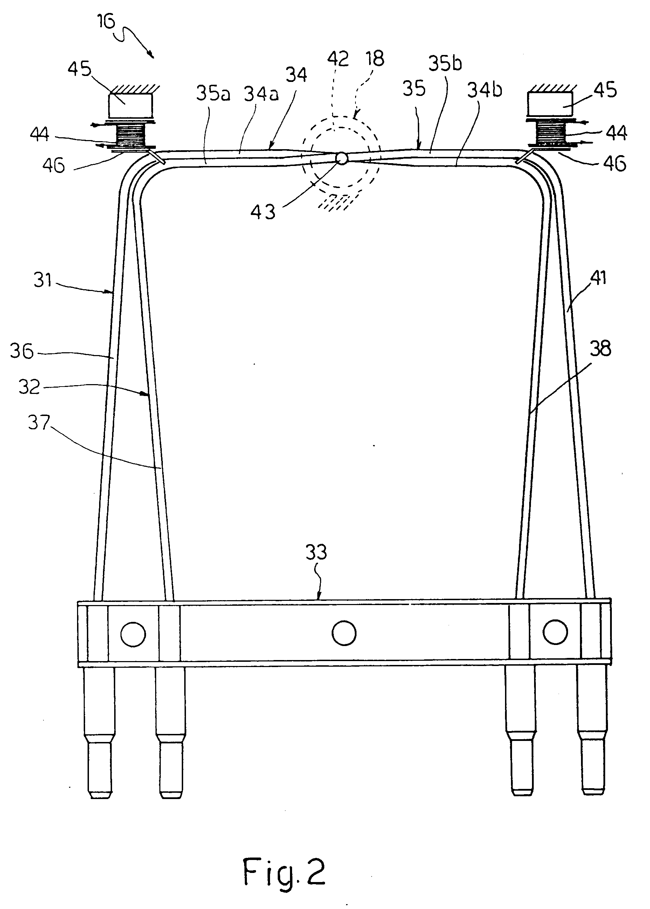

For a better understanding of the present invention a differential flow meter having a structure as described in the Italian Industrial Utility model application filed 8 February 1990 under Application No 4721 B/90 will now be described with reference to Figure 2. The flow meter 16 comprises two tubes 31 and 32 through which flow, in countercurrent, two flows φ1 and φ2 and is provided with means for detecting the mass flow rate difference (grammes/minute) of the two flows φ1 and φ2. The tubes 31 and 32 are of small diameter, of inverted U-shape and are fixed to a base body 33 in which the hydraulic connections between these and respective ducts are formed. The tubes 31 and 32 lie in the same plane and are identical to one another in length. Moreover, the tubes 31 and 32 have the same shape with respect to the direction of the flow which passes through them. The tube 31 has a central portion 34 parallel to the longitudinal axis of the body 33 but defined by two parallel rectilinear parts 34a and 34b, of the same longitudinal extent but at a different distance from the body 33. The tube 32 has a central portion 35 parallel to the portion 34, of the same longitudinal extent and also defined by two parallel rectilinear parts 35a and 35b, of the same longitudinal extent but at a different distance from the body 33. The part 35a lies at a lower level than that of the part 35b and in particular lies immediately beneath the part 34a and coaxial with the part 34b. The part 35b lies above the part 34b and coaxial with the part 34a. There is thus defined a crossover between the portions 34 and 35 in their intermediate zone at which there is formed a fixing weld.

From each portion 34 and 35 extend two rectilinear lateral arms which are fixedly joined, by welding, to the base body 33. From the parts 34a and 35a extend respective arms 36 and 37, whilst from the parts 34b and 35b extend respective arms 38 and 41. The arm 36 defines an obtuse angle with the part 34a whilst the arm 37 defines an acute angle with the part 35a. This acute angle is supplementary to the obtuse angle just mentioned, that is to say their sum is an angle of 180°. The arm 38 defines an acute angle with the part 34b equal to that defined between the arm 37 and the part 35a, whilst the arm 41 defines an obtuse angle with the part 35b equal to that defined between the arms 36 and the part 34a.

Sections of the ducts mentioned during the description of Figure 1 are connected by hydraulic couplings to the arms 36, 37, 38 and 41 at the base body. In particular, the arm 36 is connected to an initial section 6a of the duct 6 in which the pump 12 is installed, the arm 38 is connected to a second section 6b of the duct 6 in which the filter 26 is installed, the arm 41 is connected to a first section 7a of the duct 7 in which the pump 13 is installed, and the arm 37 is connected to a second section 7b of the duct 7 which carries the fluid towards a discharge not illustrated.

The flow meter 16 includes an electric device operable to cause oscillation at the resonant frequency of the system defined by the tubes 31 and 32 about an axis defined by linking the points where the arms 36, 37, 38 and 41 meet the base body 33. The said electrical device includes the electromagnet 18 having an electrical winding 41 fixed to a fixed body, and a permanent magnet 43. This is a bar magnet and is fixed by welding or soldering to the portions 34 and 35 at their crossover point. The magnet 43 has an axis orthogonal to the portions 34 and 35 and parallel to the body 33.

The flow meter 16 is provided with a second electrical device operable to generate an electrical signal proportional to the angle of inclination of the portions 34 and 35 with respect to a reference axis defined by these and relative to an oscillation of the tubes 31 and 32 when the same mass flow rate flows in them. This second device includes two electrical coils 44 each of which is coupled to a permanent magnet 45. The flow meter 16 includes two brackets 46 one fixed to the parts 34a and 35a at a first end of the portions 34 and 35, and the second fixed to the parts 34b and 35b at a second end, opposite the first, of the portions 34 and 35. Each bracket 46 supports one of the coils 44 which therefore oscillates together with the system. In a manner not illustrated the magnets 45 are fixed to a fixed body.

From what has been described the numerous advantages consequent on the present invention are evident.

In particular, the apparatus utilises a differential flow meter which evaluates the difference between the quantity of fresh dialysing liquid, the sum of that sent to the dialyser and that infused into the patient's blood, and the quantity of fluid extracted from the dialyser the sum of the liquid delivered to the dialyser, the water and metabolic wastes subtracted from the patient, and which relate to the patient's weight loss, and a quantity of water equal to the quantity of infused liquid. In substance, with the use of the differential flow meter there is obtained an absolute certainty of subtraction with extreme precision, together with a quantity relating only to the patient's weight loss, a quantity equal to that of the infused liquid. Consequently, even if the quantity to be infused is set in an imperfectly precise manner or even if the infused quantity, due to errors in the peristaltic pump, is not equal to the programmed quantity, the quantity of infused liquid does not influence the patient's weight loss in that the exact quantity infused is subtracted in any event. The apparatus of the present invention, not being provided with flow measuring devices is thus of smaller dimensions, simple construction and generally is tolerant of errors in these devices.

A further but nonetheless important advantage of the apparatus of the present invention lies in the fact that the infused liquid is constituted by the fresh dialysing liquid which, as is known, is prepared from time to time for each dialysing session. Consequently the disadvantages which are encountered by utilising the usual infusion bags are overcome at a stroke; for example the need for a balance for detecting the infused quantity, the fact that the liquid to be infused is prepared before hand and therefore can be subject to degradation, and the storage of such bags which requires labour and appropriate space are all overcome.

Finally, it is clear that the apparatus as described and illustrated here can be made with modifications and variations without by this departing from the protective ambit of the present invention.

## Claims

1. Apparatus for hemodiafiltration treatment comprising:
a dialyser (2) having a membrane (3) which separates a first circuit in which a patient's blood circulates from a second circuit in which the dialysing liquid circulates, the first circuit having a first or input duct (4) in which a first pump (11) is installed, and a second or output duct (5);
a third duct (6) forming part of the said second circuit and leading to the said dialyser (2), and in which a second pump (12) is installed, a first fluid (φ1) circulating in said third duct (6) and being constituted by the sum of a first quantity of fresh dialysing liquid delivered to the said dialyser (2) and a second quantity of fresh dialysing liquid to be infused into the patient's blood;
a fourth duct (7) forming part of the said second circuit and extending from the said dialyser (2), and in which a third pump (13) is installed to extract from the said dialyser (2) a second fluid (φ2) constituted by sum of said first quantity and a third quantity of liquid extracted from the patient's blood formed of water and metabolic waste; and
a fifth duct (8) in which a fourth pump (14) is installed, the said fifth duct (8) branching from the said third duct (6) to delivery the said second quantity of fresh dialysing liquid into the patient's blood;
characterised by the fact that it includes:
a differential flow meter (16) having first and second substantially U-shaped tubes (31, 32) of the same shape and longitudinal extent, lying into the same plane and traversed respectively by the said fluids (φ1, φ2) in countercurrent, the first tube (31) being installed between two parts (6a and 6b) of the said third duct (6), which are located between the said second pump (12) and the branching point of the said fifth duct (8), and the second tube (32) being installed between two parts (7a and 7b) of the said fourth duct (7) downstream of the said third pump (13), the said fifth duct (8) directly joining the said second duct (5);
an electric unit (17) in the said differential flow meter (16), operable to generate an electric signal proportional to the difference in mass flow rate between the said fluids (φ1, φ2) flowing through the said tubes (31 and 32); and
an electronic central control unit (21) operable to process the signal generated by the said electric unit (17) and to control the said third pump (13) to extract from the said dialyser the said second fluid (φ2), the said third quantity of liquid being related to the programmed weight loss of the patient, which value is set in the said central control unit (21), and to a quantity of water equal to the said second quantity.

2. Apparatus according to Claim 1, characterised by the fact that on the basis of data set into said central control unit (21) and relating to the said first quantity and to the said second quantity, the said central control unit (21) controls the said second and fourth pumps (12 and 14) in such a way that along the said first tube (31) of the said flow meter (16) flows the said sum of said first and second quantities.

3. Apparatus according to any one of the preceding Claims, characterised by the fact that the said central control unit (21) controls the said first pump (11) on the basis of the data set therein relating to the quantity of blood to be put into circulation into the said dialyser (2).

4. Apparatus according to any one of the preceding Claims, characterised by the fact that on the basis of the set value of the patient's weight loss, programmed into the said central control unit (21), if the electric signal generated by the said electric unit (17) is of a value different from the said set value, the said central control unit (21) controls the said third pump (13) so that along the said second tube (32) of the said flow meter (16) flows said sum of the said first quantity and of the said third quantity of the said second fluid (φ2).

5. Apparatus according to at least one of the preceding Claims, characterised by the fact that it includes a first filter (26) installed in the said third tube (6) between the said flow meter (16) and the branching point of the said fifth duct (8).

6. Apparatus according to at least one of the preceding Claims, characterised by the fact that it includes a second filter (27) installed in the fifth duct (8).

7. Apparatus according to at least one of the preceding Claims, characterised by the fact that the said first and fourth pumps (11, 14) are of peristaltic type.

## Patentansprüche

1. Gerät zur Hämodialysefiltrationsbehandlung, welches umfaßt:
einen Dialysator (2) mit einer Membran (3), welche einen ersten Kreislauf, in welchem das Blut eines Patienten zirkuliert, von einem zweiten Kreislauf trennt, in welchem die Dialyseflüssigkeit zirkuliert, wobei der erste Kreislauf eine erste oder Eingangsleitung (4), in welcher eine erste Pumpe (11) installiert ist, und eine zweite oder Ausgangsleitung (5) hat;
eine dritte Leitung (6), welche einen Teil des zweiten Kreislaufs bildet und zu dem Dialysator (2) führt, und in welcher eine zweite Pumpe (12) installiert ist, wobei eine erste Flüssigkeit (φ1) in der dritten Leitung (6) zirkuliert, welche aus der Summe einer ersten Menge von frischer, dem Dialysator (2) zugeführter Dialyseflüssigkeit und einer zweiten Menge von frischer, als Infusion in das Blut des Patienten zu gebender Dialyseflüssigkeit besteht;
eine vierte Leitung (7), welche einen Teil des zweiten Kreislaufs bildet und sich von dem Dialysator (2) weg erstreckt, und in der eine dritte Pumpe (13) installiert ist, um dem Dialysator (2) eine zweite Flüssigkeit (φ2) zu entnehmen, welche aus der Summe der ersten Menge und einer dritten Menge von aus dem Blut des Patienten entzogener und von Wasser und Stoffwechselabfallprodukte gebildeter Flüssigkeit besteht; und
eine fünfte Leitung (8), in welcher eine vierte Pumpe (14) installiert ist, wobei die fünfte Leitung (8) von der dritten Leitung (6) abzweigt, um die zweite Menge von frischer Dialyseflüssigkeit in das Blut des Patienten abzugeben;
dadurch gekennzeichnet, daß es umfaßt:
einen Differentialdurchflußmesser (16) mit einem ersten und einem zweiten, im wesentlichen U-förmigen Rohr (31, 32) mit der gleichen Form und Längserstreckung, welche in der gleichen Ebene liegen und jeweils gegenläufig von den Flüssigkeiten (φ1, φ2) durchströmt werden, wobei das erste Rohr (31) zwischen zwei Teilstücken (6a und 6b) der dritten Leitung (6) installiert ist, welche zwischen der zweiten Pumpe (12) und dem Abzweigepunkt der fünften Leitung (8) angeordnet sind, und wobei das zweite Rohr (32) zwischen zwei Teilstücken (7a und 7b) der vierten Leitung (7) stromabwärts von der dritten Pumpe (13) installiert ist, wobei die fünfte Leitung (8) unmittelbar in die zweite Leitung (5) einmündet;
eine elektrische Einheit (17) in dem Differentialdurchflußmesser (16) zum Erzeugen eines elektrischen Signals, welches proportional zu der Differenz des jeweiligen Massendurchsatzes der durch die Rohre (31 und 32) fließenden Flüssigkeiten (φ1, φ2) ist; und
eine elektronische Zentralsteuereinheit (21) zur Verarbeitung des durch die elektrische Einheit (17) erzeugten Signals und zur Steuerung der dritten Pumpe (13), um dem Dialysator die zweite Flüssigkeit (φ2) zu entnehmen, wobei die dritte Menge Flüssigkeit in Beziehung zu dem programmierten Gewichtsverlust des Patienten steht und der Wert in der Zentralsteuereinheit (21) eingestellt ist, sowie zu einer Menge von Wasser, die gleich der zweiten Menge ist.

2. Gerät nach Anspruch 1, dadurch gekennzeichnet, daß die Zentralsteuereinheit (21) die zweite Pumpe (12) und die vierte Pumpe (14) basierend auf den in der Zentralsteuereinheit (21) eingestellten Daten bezüglich der ersten Menge und der zweiten Menge so steuert, daß entlang des ersten Rohrs (31) des Durchflußmessers (16) die Summe der ersten und zweiten Menge fließt.

3. Gerät nach einem der vorhergehenden Ansprüche, dadurch gekennzeichnet, daß die Zentralsteuereinheit (21) die erste Pumpe (11) auf Basis der darin eingestellten Daten steuert, welche sich auf die Menge Blut bezieht, die in dem Dialysator (2) in Umlauf gesetzt werden soll.

4. Gerät nach einem der vorhergehenden Ansprüche, dadurch gekennzeichnet, daß basierend auf dem eingestellten Wert des Gewichtsverlustes des Patienten, welcher in der Zentralsteuereinheit (21) programmiert ist, falls das von der elektrischen Einheit (17) erzeugte elektrische Signal einen anderen Wert hat als den eingestellten Wert, die Zentralsteuereinheit (21) die dritte Pumpe (13) so steuert, daß entlang des zweiten Rohrs (32) des Durchflußmessers (16) die Summe der ersten Menge und der dritten Menge der zweiten Flüssigkeit (φ2) fließt.

5. Gerät nach mindestens einem der vorhergehenden Ansprüche, dadurch gekennzeichnet, daß es einen ersten Filter (26) umfaßt, welcher in dem dritten Rohr (6) zwischen dem Durchflußmesser (16) und dem Abzweigepunkt der fünften Leitung (8) installiert ist.

6. Gerät nach mindestens einem der vorhergehenden Ansprüche, dadurch gekennzeichnet, daß es einen zweiten Filter (27) umfaßt, welcher in der fünften Leitung (8) installiert ist.

7. Gerät nach mindestens einem der vorhergehenden Ansprüche, dadurch gekennzeichnet, daß die erste Pumpe (11) und die vierte Pumpe (14) vom peristaltischen Typ sind.

## Revendications

1. Appareil pour un traitement d'hémodiafiltration comprenant :
un dialyseur (2) ayant une membrane (3) qui sépare un premier circuit, dans lequel circule le sang d'un patient, d'un deuxième circuit dans lequel circule le liquide de dialyse, le premier circuit ayant un premier conduit on conduit d'entrée (4), dans lequel est installée une première pompe (11), et un deuxième conduit ou conduit de sortie (5) ;
un troisième conduit (6), faisant partie dudit deuxième circuit et menant audit dialyseur (2) et dans lequel une deuxième pompe (12) est installée, un premier fluide (φ1) circulant dans ledit troisième conduit (6) et étant constitué de la somme d'une première quantité de liquide de dialyse frais fourni audit dialyseur (2) et d'une deuxième quantité de liquide de dialyse frais à infuser dans le sang du patient ;
un quatrième conduit (7) faisant partie dudit deuxième circuit et s'étendant depuis ledit dialyseur (2), et dans lequel une troisième pompe (13) est installée pour extraire dudit dialyseur (2) un deuxième fluide (φ2) constitué de la somme de ladite première quantité et d'une troisième quantité de liquide extraite du sang de patient, formée d'eau et de déchets métaboliques ; et
un cinquième conduit (8) dans lequel est installée une quatrième pompe (14), ledit cinquième conduit (8) se ramifiant dudit troisième circuit (6) pour amener ladite deuxième quantité de liquide de dialyse frais au sang du patient ;
caractérisé par le fait de comprendre ;
un débitmètre différentiel (16) ayant des premier et deuxième tubes (31, 32) sensiblement en forme de U, de mêmes forme et longueur, s'étendant dans le même plan et respectivement traversés par lesdits fluides (φ1, φ2) à contre-courant, le premier tube (31) étant installé entre deux parties (6a et 6b) dudit troisième conduit (6), qui sont situés entre ladite deuxième pompe (12) et le point de ramification dudit cinquième conduit (8), et le deuxième tube (32) étant installé entre deux parties (7a et 7b) dudit quatrième conduit (7), en aval de ladite troisième pompe (13), ledit cinquième conduit (8) étant directement relié audit deuxième conduit (5) ;
une unité électrique (17) prévue dans le débitmètre différentiel (16) servant à produire un signal électrique proportionnel à la différence de débit de masse entre lesdits fluides (φ1, φ2) s'écoulent dans lesdits tubes (31 et 32) ; et
une unité de commande centrale électronique (21) servant à traiter le signal produit par ladite unité électrique (17) et à commander ladite troisième pompe (13) pour extraire dudit dialyseur ledit deuxième fluide (φ2), ladite troisième quantité de liquide dépendant de la perte de poids programmée du patient, cette valeur étant établie dans ladite unité de commande centrale (21), et d'une quantité d'eau égale à ladite deuxième quantité.

2. Appareil selon le revendication 1, caractérisé par le fait que, d'après les données établies dans ladite unité de commande centrale (20) et concernant ladite première quantité et ladite deuxième quantité, ladite unité de commande centrale (21) commande lesdites deuxième et quatrième pompes (12 et 14), de manière que ladite somme desdites première et deuxième quantités s'écoule le long dudit premier tube (31) débitmètre (16).

3. Appareil selon l'une quelconque des revendications précédentes, caractérisé par le fait que ladite unité de commande centrale (21) commande ladite première pompe (11) d'après des données qui y sont établies concernant la quantité de sang à mettre en circulation dans ledit dialyseur (2).

4. Appareil selon l'une quelconque des revendications précédentes, caractérisé par le fait que, d'après la valeur établie de la perte en poids du patient, programmée dans ladite unité de commande centrale (21), si le signal électrique produit par ladite unite électrique (17) a une valeur différente de ladite valeur établie, ladite unité de commande centrale commande ladite troisième pompe (13) de manière que, le long dudit deuxième tube (32) dudit débitmètre (16), s'écoule ladite somme de ladite première quantité et de ladite troisième quantité dudit deuxième fluide (φ2).

5. Appareil selon l'une au moins des revendications précédentes, caractérisé par le fait qu'il comporte un premier filtre (26) installé dans ledit troisième conduit (6) entre ledit débitmètre (16) et le point de ramification dudit cinquième conduit (8).

6. Appareil selon l'une au moins des revendications précédentes, caractérisé par le fait qu'il comporte un deuxième filtre (27) installé dans le cinquième conduit (8).

7. Appareil selon l'une au moins des revendications précédentes, caractérisé par le fait que lesdites première et quatrième pompes (11, 14) sont de type péristaltique.
